# EUROPEAN PATENT SPECIFICATION

(11) **EP 1 390 022 B1**
(45) Date of publication and mention of the grant of the patent: **18.04.2007**
(21) Application number: 02771681.0
(22) Date of filing: 21.05.2002
(51) Int. Cl.: A61K 31/00, A61P 25/02, A61P 25/04

(54) **USE OF 4-(2-FLUOROPHENYL)-6-METHYL-2-(1-PIPERAZINYL)THIENO 2,3-D|PYRIMIDINE FOR THE TREATMENT OF PAIN**
Verwendung von 4-(2-Fluorophenyl)-6-Methyl-2-(1-Piperazinyl) Thieno (2,3-D)Pyrimidin zur Schmerzbehandlung
NOUVEL USAGE THERAPEUTIQUE DE 4-(2-FLUOROPHENYL)-6-METHYL-2-(1-PIPERAZINYL)THIENO 2,3-D|PYRIMIDINE

(30) Priority: 22.05.2001 GB 0112494
(43) Date of publication of application: 25.02.2004
(73) Proprietor: Arachnova Therapeutics Ltd., St. Helier, Jersey JE1 1BX (GB)
(72) Inventor: BARDSLEY, Hazel Judith, Altana Pharma AG, 78467 Konstanz (DE); GRISTWOOD, Robert William, Arachnova Limited, Cambridge CB4 0WS (GB); CAVALLA, David, Arachnova Limited, Cambridge CB4 0WS (GB)
(74) Representative: Perry, Robert Edward
(86) International application number: PCT/GB2002/002388
(87) International publication number: WO 2002/094249

(56) References cited:
- EP-A- 0 150 469

## Description

### Field of the Invention

This invention relates to a new therapeutic use for a known compound.

### Background of the Invention

4-(2-Fluorophenyl)-6-methyl-2-(1-piperazinyl)thieno [2, 3-D]pyrimidine monohydrate hydrochloride is known (see US-A-4695568) and has shown activity as an antidepressant. It has serotonin and noradrenergic reuptake-blocking properties and these may be the mechanism of its action as an antidepressant. The compound also has 5HT-3 blocking activity.

Pain can be characterised as mild, moderate or severe. It can be acute or chronic in nature. Acute pain tends to resolve within minutes or days but if it persists beyond two weeks or so it is often termed chronic. Pain can be due to trauma or inflammation, and this is often termed nociceptive pain. However, pain not simply due to these causes but due mainly to nerve dysfunction or dysfunctional processing of sensory impulses is often referred to as neuropathic or neurogenic pain.

Acute nociceptive pain is well managed with non-steroidal anti-inflammatory drugs (NSAIDs) such as ibuprofen and aspirin if it is mild. For moderate pain, tramadol and codeine are useful. For severe pain, opiates such as morphine work well.

Mild to moderate chronic nociceptive pain due to inflammation is usually well managed with NSAIDs and COX-2 inhibitors although these drugs can cause serious gastric ulceration and bleeding. Tramadol is also very effective but can cause nausea, vomiting and constipation. For moderate nociceptive pain, opiates like oxycodone and codeine are useful but they cause constipation. For severe nociceptive pain, opiates such as morphine are the mainstay of treatment despite the risk of respiratory depression and addiction.

Few adequate treatments exist for neuropathic pain, and only gabapentin is licensed for this purpose. New medicines are urgently required for neuropathic pain. There is also a need for safer and stronger analgesics for nociceptive pain.

### Summary of the Invention

Surprisingly, it has been found that the known compound identified above (referred to herein as MCI-225) has activity in the treatment of pain. Its combination of serotonin and noradrenergic reuptake blockade and 5HT-3 receptor blockade has not previously been identified as being responsible for activity in pain. It will be appreciated that any suitable form of the active principle may be used, e.g. another salt form.

### Description of the Invention

By means of this invention, pain can be treated, e.g. controlled or prevented. For this purpose, the active compound can be formulated in any suitable manner together with a conventional diluent or carrier. The active compound is preferably administered by the oral route; other suitable routes of administration include sublingual/buccal, transdermal, intramuscular, intranasal, rectal, parenteral, subcutaneous, pulmonary and topical. An effective dose of the active agent will depend on the nature and degree of the complaint, the age and condition of the patient and other factors known to those skilled in the art. A typical daily dosage may be 0.1 mg to 5 mg.

A pharmaceutical composition containing the active ingredient may be in the form of a sublingual tablet or patch. Suitable compositions for oral use include tablets, troches, lozenges, aqueous or oily suspensions, dispersible powders or granules, emulsions, hard or soft capsules, syrups and elixirs. Suitable additives include sweetening agents, flavouring agents, colouring agents and preserving agents. Tablets contain the active ingredient in admixture with non-toxic pharmaceutically acceptable excipients, e.g. inert diluents such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyceryl distearate may be employed. They may also be coated, to form osmotic therapeutic tablets for controlled release. Hard gelatin capsules may include an inert solid diluent, for example calcium carbonate, calcium phosphate or kaolin; soft gelatin capsules may include water or an oil medium, for example peanut oil, liquid paraffin or olive oil.

The following study has shown the analgesic activity of MCI-225 at an oral dose of 30mg/kg, in an *in vivo* model of inflammatory pain. The effect was comparable to that of indomethacin (1mg/kg), an NSAID widely used for chronic pain such as arthritic pain.

### Study

Three groups of rats received vehicle, indomethacin or MCI-225. There were 13 rats in each group. Inflammatory pain was induced following a modified Randall Selitto method and the pain threshold of the inflamed paw was measured using a paw pressure analgesiometer. The threshold for paw withdrawal was measured in grams at 1 and 3 hours post dose. The results are shown in the following Tables.

| Group | Treatment | Dose (mg/kg) | Group mean (± sd) pain threshold (g) of inflamed paw at: | | | |
|---|---|---|---|---|---|---|
| | | | 1 hour pre-dose | 2 hour pre-dose | 1 hour post-dose | 3 hour post-dose |
| 1 | Vehicle | 0 | 191.5 | 146.5 | 135.0 | 135.0 |
| | | | ±88.56 | ±28.82 | ±36.23 | ±34.10 |
| 2 | MCI-225 | 30 | 147.7 | 138.5 | 170.8* | 205.4** |
| | | | ±65.91 | ±34.72 | ±39.47 | ±68.30 |
| 3 | Indomethacin | 1 | 166.2 | 144.2 | 200.8* | 210.0* |
| | | | ±68.32 | ±41.32 | ±82.96 | ±107.12 |

| Group | Treatment | Dose (mg/kg) | Group mean changes (± sd) in pain threshold (g) from pre-dose reading at: | |
|---|---|---|---|---|
| | | | 1 hour post-dose | 3 hour post-dose |
| 1 | Vehicle | 0 | -11.5 | -11.5 |
| | | | ±50.56 | ±38.32 |
| 2 | MCI-225 | 30 | 32.3* | 66.9** |
| | | | ±56.41 | ±65.85 |
| 3 | Indomethacin | 1 | 56.5** | 65.8* |
| | | | ±56.18 | ±95.17 |

| | | | | |
|---|---|---|---|---|
| sd = Standard deviation | | | | |

Statistical significance of difference from vehicle-treated group: **p*<0.05, ***p*<0.01

The results show that MCI-225 was able to increase the pain threshold by 32.3 g at 1 hour and 66.9 g at 3 hours. There is a statistically significant difference between these values and those for vehicle at the same time intervals. On this basis, MCI-225 is useful for the treatment of inflammatory and other pain.

## Claims

1. Use of 4-(2-fluorophenyl)-6-methyl-2-(1-piperazinyl)thieno[2,3-D]pyrimidine or a salt thereof for the manufacture of a medicament for the treatment of pain.

2. Use according to claim 1, wherein the salt is the monohhydrate hydrochloride.

3. Use according to claim 1 or claim 2, wherein the pain is nociceptive pain.

4. Use according to claim 1 or claim 2, wherein the pain is neuropathic pain.

5. Use according to any preceding claim, wherein the 4-(2-fluorophenyl)-6-methyl-2-(1-piperazinyl)thieno[2,3-D]pyrimidine or a salt thereof is to be administered in a dose of 0.1 to 5 mg.

6. Use according to claim 5, wherein the dose is 5 mg.

7. Use according to any preceding claim, wherein the medicament is adapted for oral administration..

8. A pharmaceutical composition in unit dosage form for oral administration comprising 5 mg of 4-(2-fluorophenyl)-6-methyl-2-(1-piperazinyl)thieno[2,3-D]pyrimidine or a salt thereof.

## Patentansprüche

1. Verwendung von 4-(2-Fluorphenyl)-6-methyl-2-(1-piperazinyl)thieno[2,3-D]-pyrimidin oder einem Salz desselben für die Herstellung eines Medikaments zur Behandlung von Schmerzen.

2. Verwendung nach Anspruch 1, bei der das Salz das Monohydrat-Hydrochlorid ist.

3. Verwendung nach Anspruch 1 oder Anspruch 2, bei der die Schmerzen nozizeptive Schmerzen sind.

4. Verwendung nach Anspruch 1 oder Anspruch 2, bei der die Schmerzen neuropathische Schmerzen sind.

5. Verwendung nach irgendeinem vorangehenden Anspruch, bei der das 4-(2-Fluorphenyl)-6-methyl-2-(1-piperazinyl)thieno[2,3-D]pyrimidin oder ein Salz desselben in einer Dosis von 0,1 bis 5 mg zu verabreichen ist.

6. Verwendung nach Anspruch 5, bei der die Dosis 5 mg beträgt.

7. Verwendung nach irgendeinem vorangehenden Anspruch, bei der das Medikament für die orale Verabreichung angepasst ist.

8. Pharmazeutische Zusammensetzung in Dosierungseinheitsform für die orale Verabreichung, umfassend 5 mg 4-(2-Fluorphenyl)-6-methyl-2-(1-piperazinyl)thieno[2,3-D]pyrimidin oder eines Salzes desselben.

## Revendications

1. Usage de 4-(2-fluorophényl)-6-méthyl-2-(1-pipérazinyl)thiéno[2,3-D]pyrimidine ou d'un sel de celle-ci, pour la fabrication d'un médicament pour le traitement de la douleur.

2. Usage selon la revendication 1, dans lequel le sel est le chlorhydrate monohydraté.

3. Usage selon la revendication 1 ou la revendication 2, dans lequel la douleur est une douleur nociceptive.

4. Usage selon la revendication 1 ou la revendication 2, dans lequel la douleur est une douleur neuropathique.

5. Usage selon l'une quelconque des revendications précédentes, dans lequel la 4-(2-fluorophényl)-6-méthyl-2-(1-pipérazinyl)thiéno[2,3-D]pyrimidine ou un sel de celle-ci, est destiné à être administré à une dose de 0,1 à 5 mg.

6. Usage selon la revendication 5, dans lequel la dose est de 5 mg.

7. Usage selon l'une quelconque des revendications précédentes, dans lequel le médicament est adapté pour une administration orale.

8. Composition pharmaceutique sous la forme d'une unité posologique pour administration orale, comprenant 5 mg de 4-(2-fluorophényl)-6-méthyl-2-(1-pipérazinyl)-thiéno[2,3-D]pyrimidine ou d'un sel de celle-ci.
